# EUROPEAN PATENT SPECIFICATION

(11) **EP 1 248 788 B1**
(45) Date of publication and mention of the grant of the patent: **27.10.2004**
(21) Application number: 00985697.2
(22) Date of filing: 28.12.2000
(51) Int. Cl.: C07D 493/04

(54) **SYNTHESIS OF ISOSORBIDE MONONITRATE**
DIE SYNTHESE VON ISOSORBID MONONITRAT
SYNTHESE DE MONONITRATE D'ISOSORBIDE

(30) Priority: 29.12.1999 GB 9930778
(43) Date of publication of application: 16.10.2002
(73) Proprietor: Clariant Life Science Molecules (UK) Ltd, Horsforth, Leeds LS18 4RP (GB)
(72) Inventor: MARSTON, Richard Waldron, Worcester, Worcestershire (GB); QUIGLEY, Paul Finbar, Chester, Cheshire CH4 8BQ (GB); BROWN, Christopher Martin, Warrington, Cheshire WA5 5UT (GB); ROBERTS, Stanley Michael, Neston, Cheshire CH64 6RN (GB); CROSS, Simon John, Chester, Cheshire CH1 4BN (GB)
(74) Representative: Coleiro, Raymond
(86) International application number: PCT/GB2000/004996
(87) International publication number: WO 2001/049692

(56) References cited:
- EP-A- 1 038 862
- US-A- 4 381 400
- US-A- 4 713 466
- DE LUCCHI ET. AL: "Chemoselective Reduction of Isosorbide 2,5-dinitrate" GAZZETTA CHIMICA ITALIANA, vol. 117, no. 3, March 1987 (1987-03), pages 173-6, XP000984806 cited in the application
- K. S. RAVIKUMAR ET. AL.: "Highly Chemoselective Reduction of 2,5-Dinitro-1,4:3,6-dianhydro-D-glucitol with Titanium (III) Tetrahydroborates: Efficient Synthesis of Isomerically Pure 2- and 5-Nitro-1,4:3,6-dianhydro-D-glucitols." SYNTHESIS, vol. 1994, no. 10, October 1994 (1994-10), pages 1032-4, XP002162620
- D. BHAR ET. AL.: "A highly chemoselective reduction of isosorbide 2,5-dinitrate mediated by tetrathiomolybdate. " INDIAN JOURNAL OF CHEMISTRY, SECTION B,, vol. 36B, no. 9, September 1997 (1997-09), pages 793-5, XP000984651 cited in the application
- C. BROWN ET. AL.: "New preparative routes to isosorbide 5-mononitrate." JOURNAL OF THE CHEMICAL SOCIETY, PERKIN TRANS. I, , no. 12, 25 May 2000 (2000-05-25), pages 1809-10, XP000984703

## Description

This invention relates to the synthesis of chemical compounds, in particular the synthesis of isosorbide-5-mononitrate and substituted analogues thereof. More specifically, the invention relates to the preparation of isosorbide-5-mononitrate via selective chemical reduction of isosorbide dinitrate.

Isosorbide (1,4:3,6-dianhydro-D-glucitol) is one of the hexitol class of bicylic heterocyles derived from simple sugars, which in recent years has attracted increasing interest, particularly as regards its biological activity. This growing importance particularly of certain analogues of isosorbide is attributable to the reactivity of the hydroxyl groups, which as shown in the structural formula of isosorbide below are, relative to the ring juncture, exo at the 2-position and endo at the 5-position:

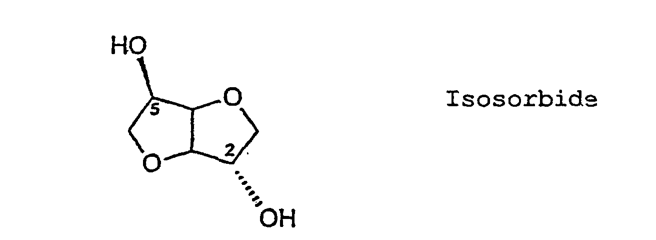

Incidentally, although omitted from the formulae throughout (for ease of drawing), the respective hydrogen atoms at the 3- and 4- positions also display chirality (each assumes an exo configuration); see Chem. Abs. Regn. No. 652-67-5.

The other two main compounds of the hexitol class, which differ from isosorbide solely in the stereochemistry of the hydroxyl groups, are isoidide (1,4:3,6-dianhydro-L-iditol) in which the hydroxyl groups are both exo relative to the ring juncture, and isomannide (1,4:3,6-dianhydro-D-mannitol) in which the hydroxyl groups both adopt an endo conformation.

The nature of the *endo* 2-hydroxyl group of isosorbide leads to internal hydrogen bonding between this hydroxyl group and the opposite ether ring oxygen. This increases the reactivity of the 5-*endo* position, thus leading to an interesting synthetic problem to the protection of the 2-position.

Isosorbide-5-mononitrate is currently the most commercially important analogue of isosorbide, which is an important vasodilator useful in cardiac treatment, for example for treating angina.

Various synthetic routes to isosorbide-5-mononitrate are known, but to date all these preparative methods suffer from disadvantages, whether it be expense, excessive time of reaction, low selectivity, safety or low yield.

For example, Seemayer et al in Tetrahedron Asymmetry, 1992, 3, 1123 and United States Patent US-A-5538891 describe the enzymatic hydrolysis of the diester analogue of isosorbide through the use of lipase from Pseudomonas fluorescens. Isosorbide is initially diacetylated using acetic anhydride and pyridine and the resulting di-acetate is then treated with the enzyme Pseudomonas fluorescens to give the 2-monoacetate. After nitration at the 5-position with nitric acid and acetic anhydride and deprotection of the 2-position with potassium carbonate and methanol, the 5-mononitrate is obtained. This however is a four-stage process involving an inefficient route to protection of the 2-position.

The bioconversion of isosorbide dinitrate has been studied by Ropena et al, as reported in Appl. Microbial. Biotechnol, 1988, 27, 358. The study identified two Cunninghamella strains for effecting chemo-selective reduction of the dinitrate and good selectivity towards the preparation of either isosorbide-2-mononitrate or isosorbide-5-mononitrate is reported.

The literature also contains disclosures of selective alkylations of isosorbide. The most productive of these methods with respect to yield is reported by Stoss et al, in Synthesis Com., 1987, 174 and European Patent application EP-A-0057847. The preparation involves acetylation with acetic anhydride followed by distillation over alkali metal carbonate, hydroxide or methylate, or lead oxide, yielding up to 70% of the desired product. However, from a practical point of view and in particular an industrial viewpoint, the use of large scale distillation apparatus can incur excessive capital expenditure. A more practical solution to this problem is reported by Cetovic et al, in Synthesis, 1989, 610. This method selectively esterifies isosorbide with carboxylic acid derivatives in the presence of 4-dimethylaminopyridine (DMAP). The yields for the functionalised isosorbide were of the order of 68 to 85% of the 2-protected diol. This approach is an example of the utilisation of the sterically unhindered 2-*exo* hydroxyl group which is esterified in preference to the more sterically crowded 5-*endo* moiety. The process however suffers from problems relating to the expense and toxicity of the reagents used.

Another approach to the selective alkylation of isosorbide is reported by Abenhaim et al in Carbohydrate Research 1994, 261, 255. Under direct alkylation using allyl, propyl and benzyl protecting groups, 40% yields of the 2-*exo*-benzyl derivative was reported. This reference also disclosed results of selective mono-acylation of isosorbide and it also suggests the use of a counter-ion for guiding the selectivity of the reaction towards the 2-*exo* moiety.

In Bull. Soc. Chim. Fr., 1988, 3, 567, Lem et al report the protection of the 2-hydroxyl function of isosorbide, preliminary dialkylation of the carbohydrate, followed by treatment with pivaloyl chloride, to yield the required 2-*exo* adduct. The yields for this route were reasonable, but it was not so promising as regards selectivity for the 2-*exo* product.

The preparation of isosorbide-5-mononitrate from isosorbide dinitrate is also a commercially important route. For example, in Ind. J. Chem., 1997, 36B, 793 Chandrasekaran et al disclose a process involving chemo-selective reduction of isosorbide dinitrate to its 5-mononitrate in a 70% yield using benzyltriethylammonium tetrathiomolybdate. In a previous paper from the same first author, in Synthesis, 1994, 1033, a corresponding process was achieved using a titanium borohydride complex which gave isosorbide-5-mononitrate in a 57% yield. The cost of such reduction complexes, however, makes such processes unfeasible on anything other than a laboratory scale.

In Gazzetta Chimica Italiana, 1987, 117, 173, Lucchi et al describe the preparation of isosorbide-5-mononitrate using a reductive system comprising zinc and acetic acid in an inert medium. Yields of 44% are reported. However, a drawback of this method is the need for addition of acetic acid at regular time intervals over a 24 hour period and the cost of disposal of a toxic, zinc-rich effluent. This system is also disclosed in published European Patent application EP-A-0201067, although recent investigations have shown this system as disclosed therein to be inadequate for achieving satisfactory yields of the desired 5-mononitrate product.

Catalytic hydrogenation of isosorbide-2,5-dinitrate has also been proposed in the literature for the preparation of isosorbide-5-mononitrate, for example in EP-A-0201067 mentioned above. In this disclosure palladium on carbon (10%) in the presence of nickel chloride as a selector is used, and is reported to give a 61% yield of the desired 5-mononitrate. This process however is undesirable for practical use in the manufacture of a pharmaceutical-grade product, not only because of the modest yield of the 5-mononitrate over the 2-mononitrate, but also because the necessary use of the NiCl₂ selector, this being an expensive material and nickel being highly toxic, thereby requiring special purification and waste treatment techniques.

Also, as disclosed in US-A-4381400, hydrazine derivatives have also shown some potential, but a drawback of this route is that yields favour the formation of isosorbide-2-mononitrate over isosorbide-5-mononitrate.
Another route to isosorbide-5-mononitrate from the 2,5-dinitrate is reported by Anteunis et al in Org. Mag. Res., 1971, 3, 693, and involves heating the dinitrate in a closed vessel with hydrochloric acid. However, only limited reduction yields are obtained and furthermore the process is not selective as between the 2- and 5-mononitrates.

As mentioned above, none of the above discussed known routes to isosorbide-5-mononitrate is free from one or more problems or disadvantages as regards yield, selectivity, time of reaction, safety or expense. There is therefore a need for a new synthetic route to isosorbide-5-mononitrate which represents an improvement on the prior art as discussed above.

With this object in mind, the present invention has been devised and involves selective chemical reduction of isosorbide-2,5-dinitrate using particular reducing systems hitherto not disclosed for this purpose.

Accordingly, in a first aspect the present invention provides a method of synthesising a compound of the following formula (1), 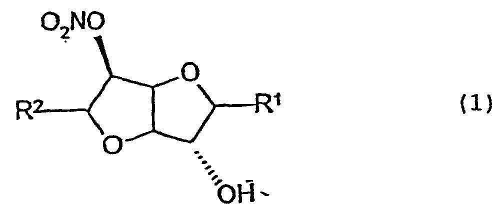 in which each of R¹ and R² is independently selected from H or optionally substituted straight or branched chain C₁-C₃₀ alkyl, C₁-C₃₀ carboxyalkyl, C₁-C₃₀ sulphoxyalkyl, C₁-C₃₀ alkoxy, C₃-C₃₀ cycloalkyl, C₃-C₃₀ carboxycycloalkyl, C₃-C₃₀ sulphoxycycloalkyl, C₃-C₃₀ cycloalkoxy, heterocyclic, carboxyheterocyclic, sulphoxyheterocyclic, oxyheterocyclic, C₃-C₃₀ cycloalkenyl, carboxycycloalkenyl, sulphoxycycloalkenyl or cycloalkenoxy, C₈-C₃₀ cycloalkynyl, carboxycycloalkynyl, sulphoxycycloalkynyl or cycloalkynoxy, C₂-C₃₀ alkynyl, carboxyalkynyl, sulphoxyalkynyl or alkynoxy group, C₄-C₃₀ aromatic, carboxyaromatic, sulphoxyaromatic or aryloxy, C₄-C₃₀ heteroaromatic, carboxyheteroaromatic, sulphoxyheteroaromatic or heteroaryloxy group, wherein in any of the said hetero atom-containing groups the hetero atom is selected from the group consisting of O, S, and N and in the case of any of the aforementioned groups being substituted, there are present one or more substituents independently selected from the group consisting of halogen, cyano, C₁-C₃₀ alkyl, C₂-C₃₀ alkenyl, C₄-C₃₀ aromatic, C₁-C₃₀ ether, C₁-C₃₀ ester, C₁-C₃₀ sulphonate ester, nitro, C₁-C₃₀ ketone, C₁-C₃₀ thioether and/or one or more pharmaceutically active groups such as 2-acetoxybenzoate, 2-N-(3'-trifluoromethylphenyl)aminobenzoate, (S)-6-methoxy-α-methyl-2-naphthaleneacetate and (S)-1-[N-[1-(ethoxycarbonyl)-3-phenylpropyl)-L-alanyl]-L-proline carboxylate; the method comprising treating a compound of the following formula (2): 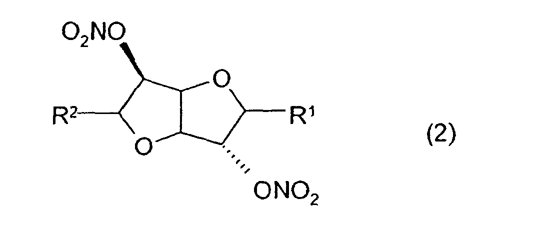 with a reducing system effective to selectively reduce the compound of formula (2) at the 2-position to produce the compound of formula (1), wherein the reducing system is selected from (i) hydrogen in the presence of a platinum oxide catalyst, or (ii) a hydride source in the presence of a transition metal phthalocyanine or polyphthalocyanine in which the transition metal is iron and/or cobalt.

In a second aspect the invention provides a compound according to formula (1) as defined above as or when produced by the method of the first aspect of the invention.

The invention further provides, in a third aspect, the use of a reducing system selected from (i) hydrogen in the presence of a platinum oxide, or (ii) a hydride source in the presence of a transition metal phthalocyanine or polyphthalocyanine in which the transition metal is iron and/or cobalt for effecting reduction selectively at the 2-position of a compound according to formula (2) to form a compound of formula (1).

Preferred features and embodiments of the present invention in its various aspects will now be described in detail.

The compound according to formula (1) as defined above, which compound is preferably that which is prepared by the method of the invention, is preferably isosorbide-5-mononitrate, i.e. that compound in which R¹ and R² are both hydrogen.

For use in the invention the isosorbide dinitrate of formula (2) may be prepared by any known synthetic route and is available commercially. By way of example, it may be prepared by direct nitration of isosorbide in a mixture of nitric acid, acetic anhydride and acetic acid at a temperature of 5-10°C, as taught by Jackson & Hayward, Can. J. Chem., 38 (1960), 496, 497, 500.

According to the invention the reducing system which is used to reduce the 2,5-dinitrate compound of formula (2) selectively (or, more correctly, preferentially) at the 2-position is selected from (i) hydrogen in the presence of a platinum oxide catalyst, or (ii) a hydride source in the presence of a transitional metal phthalocyanine or polyphthalocyanine, which transition metal is selected from iron or cobalt. In the case of the polyphthalocyanine species, this can be phthalocyanine polymer complexed with either or both of iron or cobalt.

In the case of hydrogen in the presence of a platinum oxide catalyst as the reducing system, the reaction is preferably carried out by reacting a solution of the dinitrate compound with hydrogen gas, e.g. by stirring under a hydrogen atmosphere at e.g. room temperature and at a suitable pressure.

The pressure of the hydrogen atmosphere is preferably in the range of from about 5 to about 300 psi and the reaction time is preferably from about 60 to about 600 minutes.

When hydrogen in the presence of a platinum oxide catalyst is used as the reducing system, the reaction may be carried out further in the presence of a not easily oxidisable coordinating metal salt, in order to assist the selectivity of the reduction process towards the 2-position on the isosorbide moiety. A suitable such coordinating metal salt is nickel (II) chloride. It may however be desirable not to use such a material in the carrying out of the process in view of the toxicity implications of nickel, and indeed it is an advantage of the reducing systems according to the invention that such a selector, as used for example in EP-A-0201067, is not necessary for achieving adequate yields of the decided 5-mononitrate product.

If used, however, the not easily oxidisable coordinating metal salt as a selector may preferably be used in an amount of from about 0.5% to about 50% molar equivalent (based on the amount of compound of formula (2)).

In the case of hydride source/phthalocyanine as the reducing system, the hydride source acts as the principal reducing agent, with the transition metal (iron or cobalt) phthalocyanine (or polyphthalocyanine) acting as a coordination catalyst to drive the reduction selectively to the 2-position.

The hydride source may be selected from any of the following: alkali metal tetrahydroborates (e.g. sodium, potassium or lithium tetrahydroborates), alkali metal hydrides (such as sodium, potassium or lithium hydrides), alkali metal tetrahydridoaluminates (such as those of lithium or sodium), alkali metal alkyl borohydrides (where the alkali metal is lithium, sodium or potassium, and up to three alkyl groups can be present, each alkyl group comprising branched chain, linear or cyclic carbon groups of up to 20 carbon atoms)), alkali metal alkoxy borohydrides (where the alkali metal is lithium, sodium or potassium, and up to three alkoxy groups can be present, each alkoxy group comprising branched chain, linear or cyclic carbon groups of up to 20 carbon atoms)), alkali metal alkyl hydridoaluminates (where the alkali metal is lithium, sodium or potassium, and up to three alkyl groups can be present, each alkyl group comprising branched chain, linear or cyclic carbon groups of up to 20 carbon atoms)), or alkali metal alkoxyhydridoaluminates (where the alkali metal is lithium, sodium or potassium, and up to three alkoxy groups can be present , each alkoxy group comprising branched chain, linear or cyclic carbon groups of up to 20 carbon atoms).

The catalyst is preferably selected from iron or cobalt phthalocyanine, which together with the hydride source (preferably sodium tetrahydroborate) give excellent yields and degrees of selectivity in the conversion of isosorbide dinitrate to isosorbide-5-mononitrate.

The amount of hydride source (especially sodium tetrahydroborate) used in the process of the invention may typically be from about 1 to about 10 molar equivalents, more preferably from about 1.5 to about 6 molar equivalents (based on the amount of compound of formula (2)). The amount of the transition metal phthalocyanine catalyst will typically be from about 1 to about 10% molar equivalents (on the same basis).

In the methods of the invention, and as already mentioned above as being preferred in the context of the hydrogen/platinum oxide reducing system, the reaction is preferably carried out in an organic solvent. Methanol is a preferred organic solvent, although other suitable organic solvents may include higher aliphatic alcohols, chloroform, THF, dichloromethane, t-butanol, acetone, t-butylmethylether, acetonitrile or dimethylformamide. Any combination of one or more organic solvent components may be used. However, it has been found to be particularly preferred to use a solvent system comprising a mixture of organic solvents especially dichloromethane/methanol or dichloromethane/ethanol. Such organic solvent mixtures are advantageous because they permit control over the vigour of the reaction between the isosorbide dinitrate and the hydride source/transition metal phthalocyanine catalyst, whilst the methanol component assists in the dissolution of the sodium tetrahydro borate allowing better reaction with the dissolved isosorbide dinitrate. The dichloromethane/methanol solvent mixture is somewhat preferred over the dichloromethane/ethanol system, because of the lower solubility of sodium tetrahydroborate in ethanol as compared within methanol. It is possible, however, that the solvent reaction medium may also contain an amount of water, e.g. in an amount of up to about 20% by weight of the total amount of solvent.

The present invention will now be demonstrated in further detail, by way of non-limiting example only, in the following Examples.

### Examples

For the purpose of detection and characterisation of the various products of interest in the Examples below, techniques employed included any suitable combination of ¹HNMR and ¹³CNMR spectroscopy, gas chromatography (GC), high performance liquid chromatography (HPLC), elemental analysis and melting point determination. Notes about each of these techniques are set out below:

### (1) Nuclear Magnetic Resonance Spectra (NMR)

The ¹HNMR and ¹³CNMR spectra for isosorbide have been fully characterised, for example by Hopton et al, in Can. J. Chem., 1969, 47, 2395. It is noteworthy to mention that the spectrum of isosorbide is complicated with respect to the other parent compounds isomannide and isoidide, owing to the loss of symmetry arising from the endo-exo nature of the hydroxyl groups. This characteristic induces inequivalence of the hydrogéns within the two rings.

The observed ¹HNMR spectrum for isosorbide-5-mononitrate is as follows:
¹HNMR (300 MHz, CDCl₃) δ, ppm : 2.1(1H,s,OH), 3.98(4H,m), 4.37(1H,s), 4.42(1H,d,J=4.95), 5.0(1H,t,J=5.08 and 4.95), 5.35(1H,M).

### (2) Gas Chromatography (GC)

A SE-30 column was used, with the following temperature settings: Col; 160°C, inj:180°c, det:240°C.

### (3) High Performance Liquid Chromatography (HPLC)

A RP C-18 column was used; mobile phase:82:18, water:methanol; flow rate:1.1 ml/min; detector:222nm;inj.vo1:15µl.

### (4) Elemental Analysis

A Carlo Erba Strumentazione mod. 1106 CHN analyzer was used according to the standard procedure.

### (5) Melting Point Determination

A Gallenkamp (UK) electrothermal melting point unit was used according to the standard procedure.

### Example 1 (Background Example)

As a background experiment to demonstrate the effect of the different steric natures of the nitrate ester functions in isosorbide-2,5-dinitrate on the selectivity of reduction as between the 2 and 5 positions, in separate procedures isosorbide-2-mononitrate and isosorbide-5-mononitrate were subjected to reduction using hydrogen gas in the presence of platinum oxide catalyst.

Isosorbide-2-mononitrate (0.5g,2.6mmol) was dissolved in ethanol (50ml). Platinum oxide (20mg) was added and the reaction mixture stirred at room temperature. The system was de-aerated and then placed under an atmosphere of hydrogen (14 psi pressure). The reaction was monitored by HPLC for the formation of reduction products and the amount of residual isosorbide-2-mononitrate remaining in the reaction mixture.

The above procedure was repeated for isosorbide-5-mononitrate in place of the 2-mononitrate.

The results of these two experiments are illustrated in Graph 1 below.

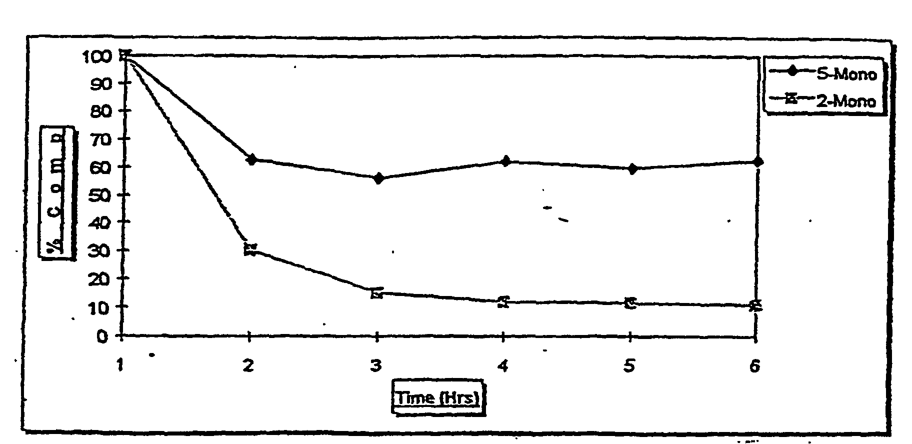

It can be seen from the above graph 1 that platinum oxide is reducing the mononitrates at different rates. Isosorbide-2-mononitrate is reduced to 10% of its initial concentration after 6 hours, but the level of isosorbide-5-mononitrate remains steady at 70% after the same time period. Without intending to be limited by theory, the reason for this display in selectivity may be attributed to the different steric nature of the 2- and 5- nitrate ester functions, attack at the 5-*endo* site being blocked by steric hindrance and preferential reduction at the 2-*exo* site occurring owing to greater accessibility.

This Example therefore demonstrates the preferential reduction of isosorbide-2,5-dinitrate at the 2-position rather than at the 5-position.

### Example 2 - Chemoselective reduction of isosorbide dinitrate using H₂/PtO₂.

This example demonstrates the use of hydrogen gas in the presence of platinum oxide catalyst as the reducing system in the method according to the invention.

Isosorbide-2,5-dinitrate (2g,8.48mmol) was dissolved in methanol (30ml). To this solution was added platinum oxide (25mg) and the air was removed using a vacuum pump and replaced with an atmosphere of hydrogen (14 p.s.i pressure). The reaction mixture was stirred at room temperature. The presence of reaction components was monitored by GC. The results are shown in Graph 2 below.

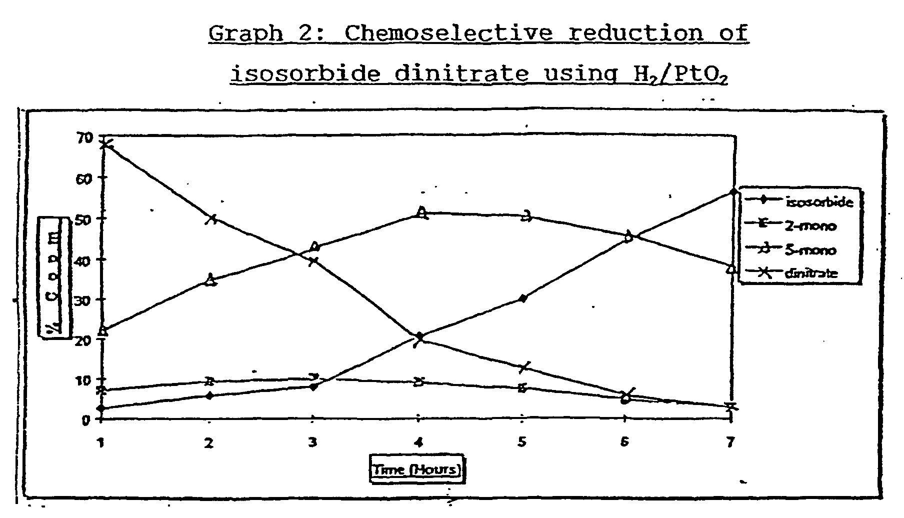

The results shown in graph 2 above confirm the nature of the selectivity of the platinum oxide catalyst towards the 2-*exo* functionality of the isosorbide moiety, producing isosorbide-5-mononitrate in moderate yields (50%).

### Example 3 - Chemoselective Reduction of Isosorbide-2,5-Dinitrate using H₂/PtO₂ with Nickel Chloride

This example demonstrates the use of H₂/PtO₂ as the reducing system, but additionally in the presence of nickel chloride as a selector to further drive the reduction towards the 2-position.

Isosorbide-2,5-dinitrate (2g,8.48mmol) was dissolved in methanol (30ml). To this solution was added platinum oxide (25mg) and nickel chloride (2g,14.9mmol). The reaction mixture was stirred at room temperature and de-aerated and then placed under an atmosphere of hydrogen (14 psi pressure). The reduction was followed by monitoring the presence of reaction components by GC. The results are shown in Graph 3 below.

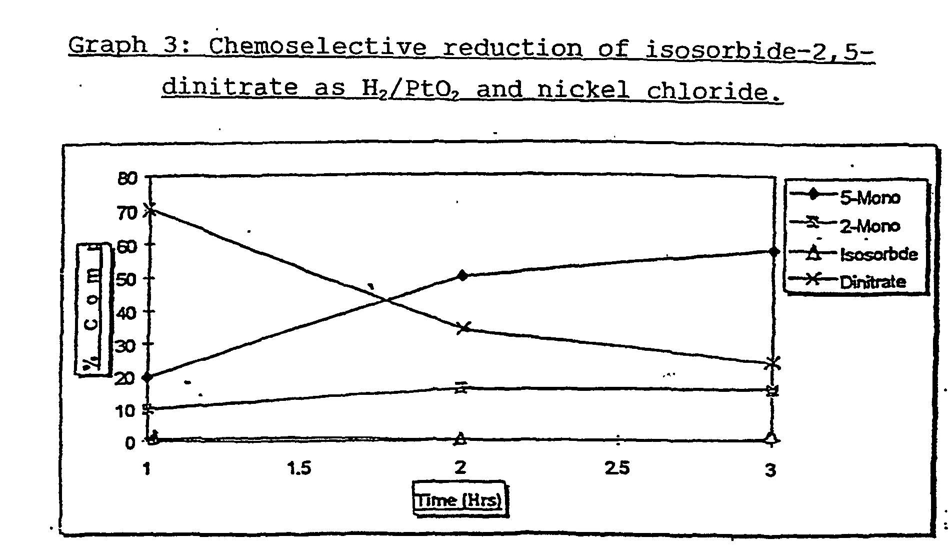

The results shown in graph 3 above displayed an increase in total yield of isosorbide-5-mononitrate by 6% after three hours. Although complete reduction had not occurred, the results show the advantageous use of nickel chloride in enhancing the selectivity of the reduction reaction. Owing to potential difficulties in extraction of nickel chloride from a practical system, it is possible that its presence may be less preferred in the case of a scaled-up industrial process.

### Example 4 - Chemoselective reduction of isosorbide dinitrate using H₂/PtO₂.

This is another example demonstrating the use of H₂/PtO₂ as the reducing system in the production and characterisation of isosorbide-5-mononitrate in accordance with a preferred embodiment of the invention.

Isosorbide-2,5-dinitrate (4g,16.94mmol) was added to methanol (80ml) and stirred at room temperature. Platinum oxide (50mg) was added and the system was de-aerated using a vacuum pump. The atmosphere was replaced with hydrogen gas (14 psi pressure) and the system was monitored by GC. After three hours the reaction was terminated by the removal of hydrogen and the catalyst was vacuum filtered over a bed of Celite. The solvent was removed under vacuum and the reduction products separated by chromatography (silica, dichloromethane:ethanol,96:4) to yield isosorbide-5-mononitrate (1.46g, 45.1%) as a white crystalline solid (m.p.90.7°C,Lit.89-91°C). The product was characterised by NMR and elemental analysis as follows:
¹HNMR (300 MHz, CDCl₃) δ, ppm:2.05(1H,OH), 3.94 (4H,m), 4.37 (1H,s), 4.41(1H,d,J=4.8), 5.00 (1H,t,J=5.3 and 5.1), 5.40 (1H,m).
¹³CNMR (300 MHz, CDCl₃) δ, ppm: 69.218, 75.732, 75.834, 81.177, 81.454, 88.907.
Elemental analysis found: % (calcd for C₁₀H₁₄O₅): C, 37.77 (37.70), H,4.73 (4.75), N,7.34 (7.33).

### Example 5 - Scale-up example using H₂/PtO₂ as the reducing system

In order to demonstrate the ability of the laboratory scale reductions exemplified above to be scaled up, the method of Example 4 was scaled up by a factor of 15.

Isosorbide-2,5-dinitrate (60g,0.254mol) was dissolved in methanol (1200ml) and platinum oxide (750mg) was added and the vessel purged with hydrogen (14 psi pressure). Once fully purged, the reaction mixture was stirred at room temperature and the pressure regulated to the desired level. The procedure was repeated for different H₂ pressures in order to further illustrate the effect of pressure on the efficiency of the reduction reaction. The experiment was repeated 5 times, using pressures of 20, 50, 70, 120 and 300 psi. The results are shown in Graph 4 below.

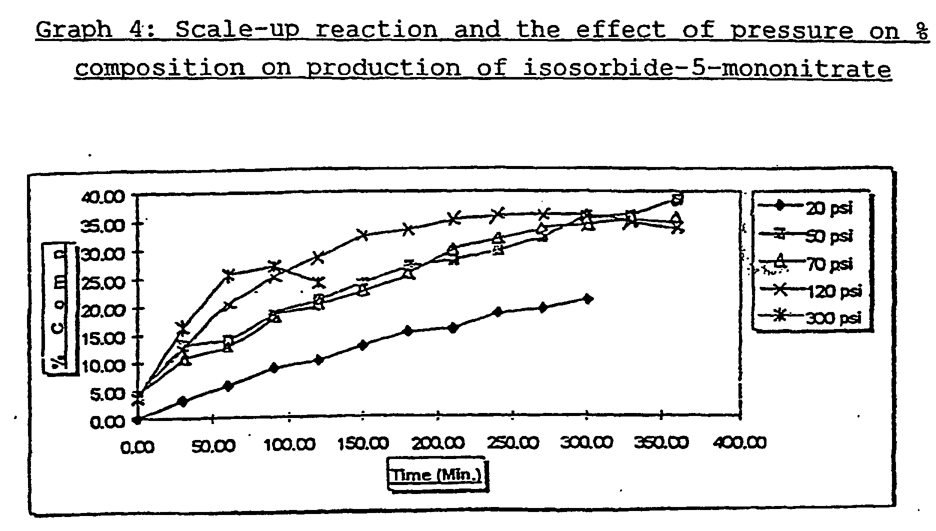

The results illustrated in graph 4 above demonstrate the ability of the H₂/PtO₂ réduction system to be scaled up with only a marginal (5%) loss of product yield. The results also show that lower pressures are preferential for the formation of isosorbide-5-mononitrate.

### Example 6 - Selective reduction of isosorbide dinitrate using sodium tetrahydroborate/iron phthalocyanine.

This example demonstrates the use of sodium tetrahydroborate/iron phthalocyanine as the reducing system in the production of isosorbide-5-mononitrate from isosorbide dinitrate in accordance with the invention.

Isosorbide dinitrate (0.500g, 2.12mmol) was dissolved in methanol (20ml) and to this solution was added iron phthalocyanine (0.075g, 0.132mmol). To this suspension was added, with stirring at room temperature, sodium tetrahydroborate (0.160g, 4.23mmol). After an initial vigorous reaction the solution was acidified with hydrochloric acid (2M HCl, 10ml), upon which the catalyst became insoluble. The solution was then neutralised by addition of sodium hydroxide (2M NaOH). The catalyst was removed by filtration and the reduction products were extracted with dichloromethane (30ml x10). Isosorbide-5-mononitrate was isolated by column chromatography (silica, acetonitrile 15% in dichloromethane). After filtration of the catalyst, it was reused in the process as detailed above. In one case the catalyst was used three times. The yield of isosorbide-5-mononitrate recovered from each use of the catalyst is shown in Table 1 below.

**TABLE 1**

| Run | Yield (%wt) |
|---|---|
| 1 | 48.1 |
| 2 | 44.2 |
| 3 | 43.1 |

This example thus also demonstrates the recyclability of the iron phthalocyanine catalyst. Given that it is expensive and that residual levels can cause colour problems in the isosorbide-5-mononitrate recovered from the process, the ability to recycle the catalyst has a major impact in terms of lowering overall raw material costs for the process. The data in the above Table 1 also show that there is little decrease in effectiveness of the catalyst after each reuse.

### Example 7 - Selective reduction of isosorbide dinitrate using sodium tetrahydroborate/cobalt phthalocyanine.

To demonstrate the use of cobalt phthalocyanine as the coordination catalyst in combination with sodium tetrahydroborate, the procedure of the initial run of Example 6 above was repeated using cobalt phthalocyanine instead of iron phthalocyanine. In five separate runs the amount of cobalt phthalocyanine was varied between 5 and 25% by weight of the isosorbide dinitrate substrate. The resulting yields of isosorbide-5-mononitrate for each run are shown in Table 2 below.

**TABLE 2**

| Run | Cobalt Phthalocyline Catalyst (%w/w) | Yield (%wt). |
|---|---|---|
| 1 | 5 | 38 |
| 2 | 10 | 42 |
| 3 | 15 | 47 |
| 4 | 20 | 41 |
| 5 | 25 | 37 |

The above results illustrate the optimum level of catalyst, peaking at around 15%w/w.

### Example 8 - Variability of cobalt phthalocyanine and sodium tetrahydroborate levels in selective reduction of isosorbide dinitrate

In this example the cobalt phthalocyanine catalyst loading (% w/w relative to isosorbide) and sodium tetrahydroborate molar equivalent levels were systematically varied. A first set of runs details the variance in isolated yield of isosorbide-5-mononitrate as the levels of cobalt phthalocyanine catalyst were varied, while holding the levels of sodium tetrahydroborate used at a constant 6 molar equivalents (relative to the level of isosorbide used). The method used was as follows:

Isosorbide dinitrate (0.5g; 2.12mmol) was dissolved in methanol (20ml) and cobalt phthalocyanine was added (0.025g, 5% w/w; 0.05g, 10% w/w; 0.075g, 15% w/w; 0.1g, 20% w/w and 0.125g, 25% w/w) at the set level to be studied. To this suspension was added.sodium tetrahydroborate (0.48g, 12.72mmol). After an initial vigorous reaction the solution was acidified (2N HCl, 10ml) and subsequently neutralised (2N NaOH). The catalyst was filtered off and the reduction products were extracted with 10x30ml portions of dichloromethane. The solvent was removed under reduced pressure and the residue was chromatographed on a silica gel column (*eluent:* acetonitrile:dichloromethane - 15:85 v/v). The isolated yields of isosorbide-5-mononitrate are detailed in Table 3 below.

**TABLE 3**

| Cobalt (%w/w) | % 5-mononitrate | Sodium borohydride |
|---|---|---|
| 5 | 38 | 6 eq |
| 10 | 42 | 6 eq |
| 15 | 47 | 6 eq |
| 20 | 41 | 6 eq |
| 25 | 37 | 6 eq |

The above results indicate that for methanol as a solvent system, a 15% catalyst loading was optimal.

In a second set of runs the system was re-examined at a 15% catalyst loading, while varying the molar equivalents of sodium tetrahydroborate used (relative to isosorbide) from 1 equivalent up to 6 equivalents. The method used for evaluation of the system was as detailed above. The isolated yields of isosorbide-5-mononitrate are detailed in Table 4 below.

**TABLE 4**

| Borohydride equivalence | % 5-mononitrate |
|---|---|
| 1 | 16.2 |
| 3 | 31.0 |
| 5 | 50.0 |
| 6 | 42.0 |

The results indicate that for this system, the usage of 5 molar equivalents of sodium tetrahydroborate was optimal.

### Example 9 (Comparative Example)

Two alternative known reduction systems, apart from the PtO₂/H₂ and sodium tetrahydroborate/transition metal phthalocyanine systems preferred for use in the invention, were tested for their ability to selectively reduce isosorbide dinitrate to the 5-mononitrate.

The systems assessed were Raney nickel/H₂ and sodium tetrahydroborate/transition metal phthalocyanine where the metal used was zinc, magnesium, sodium or vanadium. Sodium tetrahydroborate (with no metal phthalocyanine catalyst) was also evaluated as a comparative example. The experimental details were as follows:

### (a) Reduction with Raney Nickel/H₂

Isosorbide dinitrate (1g; 4.24mmol) was dissolved in methanol (200ml). Potassium hydroxide solution (0.25mol; 100ml) was added and the system was stirred at room temperature. Raney nickel (2.0g) was added to the stirred solution, portionwise, over one hour and the reaction was monitored by GC. Analysis of the mixture after addition showed >95% isosorbide content, indicating that large-scale overreduction had occurred.

### (b) Reduction with Sodium tetrahydroborate and transition metal phthalocyanine catalyst (where the metal is zinc, magnesium, sodium or vanadium)

Isosorbide dinitrate (500mg; 2.12mmol) was dissolved in methanol (20ml), and to this solution was added 75mg of the metal phthalocyanine catalyst (where the metal was zinc, magnesium, sodium or vanadium). While stirring the suspension at room temperature, sodium tetrahydroborate (160mg; 4.23mmol) was added and after an initial vigorous reaction hydrochloric acid (2M; 10ml) was added, at which point the catalyst became insoluble in the reaction mixture. The solution was neutralised by the addition of 2M sodium hydroxide solution, after which the catalyst was removed by filtration and the reduction products extracted with 10x30ml portions of dichloromethane. The dichloromethane was evaporated under reduced pressure and the residues analysed by GC. The analysis showed >95% unreacted isosorbide dinitrate.

### (c) Reduction with Sodium Tetrahydroborate only

Isosorbide dinitrate (1.0g; 4.24mmol) was dissolved in a mixture of 10ml dichloromethane and 2ml methanol,. and sodium tetrahydroborate (0.32g; 8.46mmol) was added to the stirred mixture. The mixture was stirred for a further 1 hour, then acidified with approximately 10ml of 2N HCl solution (effervescent reaction) followed by neutralisation with 2N sodium hydroxide. The organic layer was water washed and stripped under reduced pressure. GC analysis of the residue indicated 96.85% unreacted isosorbide dinitrate.

It can be seen from the above results that both of these alternative known reduction systems failed to produce the 5-mononitrate product in any significant amount, totally reducing the dinitrate to isosorbide itself or resulting in no significant reduction at all.

### Example 10 (Comparative Example)

Another known process for the selective reduction of isosorbide dinitrate to isosorbide-5-mononitrate was tested for its ability to produce the results claimed. The known process was that using zinc/acetic acid as the reduction system, as described in Gazzetta Chimica Italiana, 1987, 117, 173 (Lucchi et al) and EP-A-0201067 (Modena).

Zinc powder used in the following procedure was activated by stirring with 1% hydrochloric acid, washing with water, ethanol, acetone and diethyl ether, then dried and immediately used in the following process.

The procedure and results were as follows:

Isosorbide dinitrate (5g; 21.2mmol) was stirred in 100mls of ethanol and the mixture was cooled to -10°C under nitrogen for about 20 minutes, then zinc powder (3g; 45.9mmol; pretreated as above) and acetic acid (2ml; 46mmol) were added. After 7hrs a further 1ml of acetic acid was added. After a further 5hrs isosorbide dinitrate (3g; 13mmol), zinc powder (3g; 45.9mmol; pretreated as above) and acetic acid (2ml; 46mmol) were sequentially added. The mixture was allowed to stir for a further 10 hrs before filtration and evaporation under reduced pressure. GC analysis of the reaction mixture gave 5.4% isosorbide, 0.6% isosorbide-2-mononitrate, 55.5% isosorbide-5-mononitrate and 38.5% isosorbide dinitrate. This compares unfavourably with EP-A-201067, which claims a ratio of products comprising 15% isosorbide, 2% isosorbide-2-mononitrate, 75% isosorbide-5-mononitrate and 8% isosorbide dinitrate. Thus, this prior art process failed to give satisfactory results as regards yield of the desired 5-mononitrate product in terms of the degree to which the reaction had progressed.

### Example

This example demonstrates the use of preferred solvent mixtures for optimising the selective reduction of isosorbide dinitrate to isosorbide-5-mononitrate using sodium tetrahydroborate and iron phthalocyanine catalyst.

### a) Methanol solvent

To a stirred solution containing 200mls of methanol, 5g (21.18mmol) of isosorbide dinitrate and 0.75g (1.32mmol) of iron phthalocyanine was added, portionwise over two hours, 1.6g (42.29mmol) of sodium tetrahydroborate (granular). Successive sodium tetrahydroborate additions were made once the initial vigorous reaction had subsided. The reaction was stirred for a further 15 mins after visible effervescence had ceased. The resultant dark blue solution was filtered (to remove the catalyst), the solvent was removed under reduced pressure, and the residue was redissolved in dichloromethane. The dichloromethane solution was water washed, dried over sodium sulphate, and the solvent again removed under reduced pressure to give a residue which was analysed by GC. The results (run 1 of Table 5 below) indicated 90% selectivity of reaction towards isosorbide-5-mononitrate. The effervescence of reaction, however, would make control of sodium tetrahydroborate addition a problem on a plant scale.

### b) Ethanol solvent

A 7 molar equivalent ratio of sodium tetrahydroborate to substrate was used in this case, as detailed in the reference paper *Angew. Chem. Int*. *Ed*. *Engl*. 20, 5, (1981), which details the non-selective reduction of organic nitro compounds (rather than the selective reduction of organic nitrate esters, as is the essence of the present invention using the sodium tetrahydroborate / ethanol reduction system.

To a stirred solution containing 22mls of ethanol, 1.12g (29.6mmol) of sodium tetrahydroborate and 0.15g (0.264mmol) of iron phthalocyanine was added, portionwise, 1g (4.23mmol) of isosorbide dinitrate. Once the exothermic reaction had subsided, sufficient dilute hydrochloric acid was added to dissolve the residual boron species, the solution then being evaporated to dryness and the residue analysed by GC. The results (run 2 of Table 5 below) indicate that the large excess of sodium tetrahydroborate used has caused complete overreduction of the isosorbide dinitrate to isosorbide.

### c) Acetonitrile/ethanol solvent

Isosorbide dinitrate (1g; 4.23mmol) was dissolved in 2.5mls of acetonitrile, and this solution was added slowly to a cold (10°C) stirred solution of 0.32g (8.46mmol) of sodium tetrahydroborate and 0.15g (0.264mmol) of iron phthalocyanine in 6mls of ethanol. The resultant mixture was filtered, evaporated to dryness, then analysed by GC. The GC data (run 3 of Table 5 below) indicated essentially no remaining isosorbide dinitrate, with 84.14% isosorbide-5-mononitrate, and 13.86% isosorbide (as a result of overreduction). The run was repeated on a 5g isosorbide dinitrate scale, and the results were again very consistent with the initial run (run 4 of Table 5 below).

### d) Dichloromethane/ethanol solvent

Most of the solvents evaluated as above had shown reasonably good selectivity, but would be difficult to control on a plant scale, given the highly exothermic nature of the reduction process. A method to safely control the reduction process was therefore required and to this end it was decided to evaluate methylene chloride/alcohol mixtures where the isosorbide dinitrate would be stirred along with sodium tetrahydroborate and iron phthalocyanine catalyst in methylene chloride, in which the isosorbide dinitrate has limited solubility. By gradual addition of the alcohol the isosorbide dinitrate would gradually dissolve in the solvent mixture, and be reduced by the sodium tetrahydroborate in the presence of the iron phthalocyanine catalyst. The exothermicity of reaction could therefore be controlled by the rate of addition of the alcohol to the reaction mixture.

For evaluation of ethanol as the solvent, 1g (4.23mmol) of isosorbide dinitrate was stirred with 0.32g (8.46mmol) of sodium tetrahydroborate and 0.15g (0.264mmol) of iron phthalocyanine in 10mls of dichloromethane. Ethanol (10mls) was then gradually added to the stirred mixture to control the reaction exotherm, the reaction was monitored for completion (as evidenced by the lack of effervescence), then stirred for a further 15minutes. Acetone (5mls) was added to destroy residual sodium tetrahydroborate, then the mixture was filtered and evaporated under reduced pressure. GC analysis of the residue (run 5 of Table 5 below) showed 96.42% isosorbide-5-mononitrate, with no isosorbide dinitrate present, and only 3.43% isosorbide-2-mononitrate present (this represents an isosorbide-5-mononitrate:isosorbide-2-mononitrate ratio of 28:1, superior to any previously reported selective reduction system for isosorbide dinitrate).

The consistency of the process was demonstrated by repeated runs on the 1g isosorbide dinitrate scale using the above system (runs 6-7 of Table 5 below). In one case (run 6) an isosorbide-5-mononitrate:isosorbide-2-mononitrate ratio of 72:1 was recorded. In another case (run 7) a selectivity of 97.78% isosorbide-5-mononitrate was recorded.

### e) Dichloromethane/methanol solvent

A stirred mixture of 1g (4.23mmol) of isosorbide dinitrate, 0.32g (8.46mmol) of sodium tetrahydroborate and 0.15g (0.264mmol) of iron phthalocyanine in 10mls of dichloromethane was prepared. Methanol (2mls) was then gradually added to the stirred mixture, slowly to control the reaction exotherm, then the reaction was monitored for completion (as evidenced by the lack of effervescence), and stirred for a further 15minutes. Acetone (5mls) was added to destroy residual sodium tetrahydroborate, then the mixture was filtered and evaporated under reduced pressure. GC analysis of the residue (run 8 of Table 5 below) showed 95.11% isosorbide-5-mononitrate, with 4.67% unreacted isosorbide dinitrate, and 0.22% isosorbide-2-mononitrate. This represents an isosorbide-5-mononitrate:isosorbide-2-mononitrate ratio of 432:1.

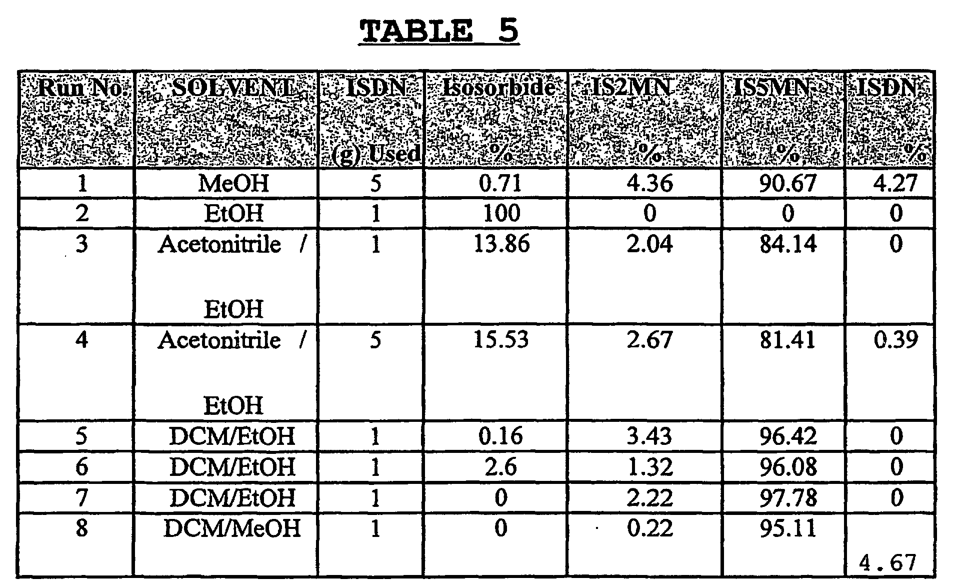

### Example 12

On the basis of the data as detailed in Example 11 above, it became clear that dichloromethane/methanol and dichloromethane/ethanol solvent compositions gave the highest selectivities of reduction of isosorbide dinitrate, and also enabled control to be exerted on the rate of reaction. The two solvent systems were selected for further process optimisation. This example describes variation in the molar quantities of sodium tetrahydroborate used for both solvent combinations.

### a) Dichloromethane/ethanol

The preparative methodology used was as described in Example 11 above. The 2 molar equivalents of sodium tetrahydroborate (as outlined in Examples 5-7 of Table 5 above) was reduced to 1.5 molar equivalents (run 1 of Table 6 below). The GC analysis of the reaction mixture (run 1 of Table 6 below) indicates that 1.5 molar equivalents of sodium tetrahydroborate is extremely undesirable for the dichloromethane/ethanol system as the reaction had only gone to 37.7% completion, with 61.85% unreacted isosorbide dinitrate remaining.

### b) Dichloromethane/methanol

The preparative methodology used was as described in Example 11 above. The 2 molar equivalents of sodium tetrahydroborate (as outlined in Example 8 of Table 5 above) was reduced to 1.75 and 1.5 molar equivalents (run 2 and 3 respectively of Table 6 below). The GC analyses indicates only a slight reduction in isosorbide-5-mononitrate content relative to the case where 2 molar equivalents of sodium tetrahydroborate were used, although it may have been the case for the use of 1.5 molar equivalents of sodium tetrahydroborate that the reaction might have progressed to give a higher overall yield of isosorbide-5-mononitrate if allowed to stir for a further hour after addition of the alcohol.

It would certainly appear from this data that it is possible to use relatively less sodium tetrahydroborate for the dichloromethane/methanol solvent system, compared with the dichloromethane/ethanol system, in order to get a good (>95%) selectivity of reduction to isosorbide-5-mononitrate.

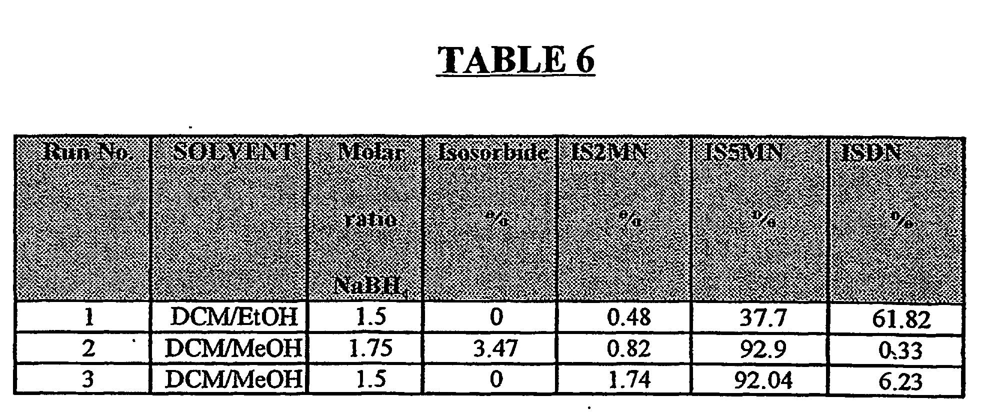

### Example 13

This example describes variation in the molar quantities of iron phthalocyanine catalyst used for both solvent combinations (dichloromethane/ethanol and dichloromethane/methanol).

### a) Dichloromethane/ethanol

The preparative methodology used was as described in Example 11 above. The 0.0623 molar equivalents of iron phthalocyanine catalyst (as outlined in Examples 5-7 of Table 5 above) was reduced by 50% to 0.0312 molar equivalents (run 1 of Table 7 below), while maintaining the sodium tetrahydroborate level at 2 molar equivalents (as for Examples 5-7 of Table 5 above). The GC analysis of the reaction mixture (run 1 of Table 7 below) indicates that 0.0312 molar equivalents of iron phthalocyanine catalyst is extremely undesirable for the dichloromethane/ethanol system as the reaction had only gone to 26.9% completion, with 70.27% unreacted isosorbide dinitrate remaining.

### b) Dichloromethane/methanol

The preparative methodology used was as described in Example 11 above. While maintaining the 1.75 molar equivalent level of sodium tetrahydroborate used (as outlined in Example 2 of Table 6 above) the iron phthalocyanine molar equivalent level was reduced from 0.0623 molar equivalents (as outlined in Example 2 of Table 6 above) to 0.0312 molar equivalents (run 2 of Table 7 below), then to 0.0125 molar equivalents (run 3 of Table 7 below), and finally to 0.0062 molar equivalents (run 4 of Table 7 below). The GC analysis of the reaction mixture (runs 2-4 of Table 7 below) indicates that halving the molar equivalent level of iron phthalocyanine catalyst level (run 2 of Table 7 below) did not have a detrimental effect on the performance of the reduction system (unlike the dichloromethane/ethanol solvent system where this level of catalyst had a very deleterious effect on yield, as per run 1 of Table 7 below). At a lower catalyst level of 0.0125 molar equivalents for the dichloromethane/methanol solvent system the overall conversion began to reduce (run 3 of Table 7 below), with 24.4% unreacted isosorbide dinitrate, while at a catalyst level of 0.0062 molar equivalents (run 4 of Table 7 below) 68.09% of unreacted isosorbide dinitrate was left upon workup of the reaction mixture. Clearly a catalyst level of 0.0312 molar equivalents is desirable for this reduction system. An experiment was also carried out using no iron phthalocyanine catalyst whatever. The results (run 5 of Table 7 below), not surprisingly, indicated almost totally unreacted isosorbide dinitrate (96.85%) after workup of the reaction. Clearly, the iron phthalocyanine catalyst has a definite role in ensuring progress of the reduction, as well as mediating a high selectivity of reduction of isosorbide dinitrate to isosorbide-5-mononitrate.

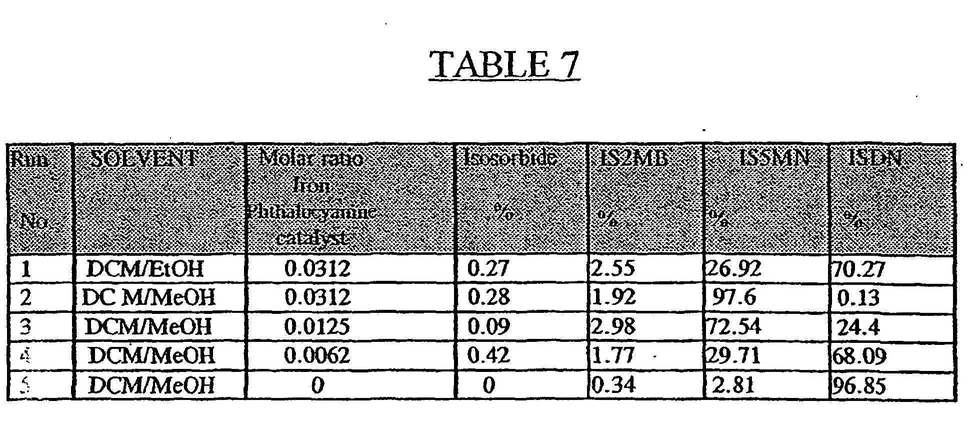

The outcome of the evaluations detailed in Examples 12 and Examples 13 above are that the dichloromethane/methanol solvent system is the desired combination, since one can utilise lower sodium tetrahydroborate and iron phthalocyanine molar equivalent levels to achieve a good yield and selectivity of isosorbide-5-mononitrate from isosorbide dinitrate, when compared with the equivalent solvent system of dichloromethane/ethanol.

## Claims

1. A method of synthesising a compound of the following formula (1), 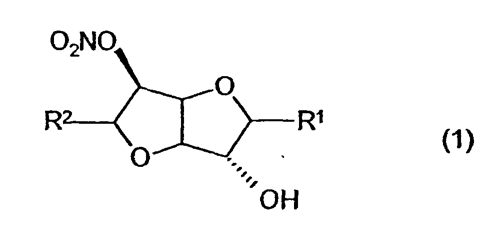 in which each of R¹ and R² is independently selected from H or optionally substituted straight or branched chain C₁-C₃₀ alkyl, C₁-C₃₀ carboxyalkyl, C₁-C₃₀ sulphoxyalkyl, C₁-C₃₀ alkoxy, C₃-C₃₀ cycloalkyl, C₃-C₃₀ carboxycycloalkyl, C₃-C₃₀ sulphoxycycloalkyl, C₃-C₃₀ cycloalkoxy, heterocyclic, carboxyheterocyclic, sulphoxyheterocyclic, oxyheterocyclic, C₃-C₃₀ cycloalkenyl, carboxycycloalkenyl, sulphoxycycloalkenyl or cycloalkenoxy, C₈-C₃₀ cycloalkynyl, carboxycycloalkynyl, sulphoxycycloalkynyl or cycloalkynoxy, C₂-C₃₀ alkynyl, carboxyalkynyl, sulphoxyalkynyl or alkynoxy group, C₄-C₃₀ aromatic, carboxyaromatic, sulphoxyaromatic or aryloxy, C₄-C₃₀ heteroaromatic, carboxyheteroaromatic, sulphoxyheteroaromatic or heteroaryloxy group, wherein in any of the said hetero atom-containing groups the hetero atom is selected from the group consisting of O, S, and N and in the case of any of the aforementioned groups being substituted, there are present one or more substituents independently selected from the group consisting of halogen, cyano, C₁-C₃₀ alkyl, C₂-C₃₀ alkenyl, C₄-C₃₀ aromatic, C₁-C₃₀ ether, C₁-C₃₀ ester, C₁-C₃₀ sulphonate ester, nitro, C₁-C₃₀ ketone, C₁-C₃₀ thioether and/or one or more pharmaceutically active groups; the method comprising treating a compound of the following formula (2): 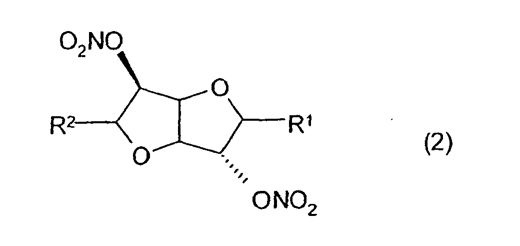 with a reducing system effective to reduce preferentially the compound of formula (2) at the 2-position to produce the compound of formula (1), wherein the reducing system comprises (i) hydrogen in the presence of a platinum oxide catalyst, or (ii) a hydride source in the presence of a transition metal phthalocyanine or polyphthalocyanine in which the transition metal is iron and/or cobalt.

2. A method according to claim 1 wherein the or each pharmaceutically active group is selected from the group consisting of 2-acetoxybenzoate, 2-N-(3'-trifluoromethylphenyl)aminobenzoate, (S)-6-methoxy-α-methyl-2-naphthaleneacetate and (S)-1-[N-[1-(ethoxycarbonyl)-3-phenylpropyl]-L-alanyl]-L-proline carboxylate.

3. A method according to claim 1 wherein R¹ and R² are both hydrogen.

4. A method according to any one of claims 1 to 3 wherein the reducing system comprises hydrogen in the presence of a platinum catalyst and the reaction is carried out at room temperature under a hydrogen gas atmosphere of 5 to 300 psi.

5. A method according to claim 3 or claim 4 wherein the reaction is carried out in a solvent comprising dichloromethane/methanol or dichloromethane/ethanol.

6. A method according to any one of claims 1 to 3 wherein the reducing system comprises a hydride source in the presence of a polyphthalocyanine polymer complexed with iron, cobalt or a mixture of both.

7. A method according to any one of claims 1 to 3 wherein the reducing system comprises a hydride source in the presence of a transition metal phthalocyanine or polyphthalocyanine in which the transition metal is iron, cobalt or a mixture of both and the hydride source is selected from the group consisting of alkali metal tetrahydroborates, alkali metal hydrides, alkali metal tetrahydridoaluminates, alkali metal alkyl borohydrides (where the alkali metal is lithium, sodium or potassium, and up to three alkyl groups can be present, each alkyl group comprising branched chain, linear or cyclic carbon groups of up to 20 carbon atoms), alkali metal alkoxy borohydrides (where the alkali metal is lithium, sodium or potassium, and up to three alkoxy groups can be present, each alkoxy group comprising branched chain, linear or cyclic carbon groups of up to 20 carbon atoms), alkali metal alkyl hydridoaluminates (where the alkali metal is lithium, sodium or potassium, and up to three alkyl groups can be present, each alkyl group comprising branched chain, linear or cyclic carbon groups of up to 20 carbon atoms), and alkali metal alkoxyhydridoaluminates (where the alkali metal is lithium, sodium or potassium, and up to three alkoxy groups can be present , each alkoxy group comprising branched chain, linear or cyclic carbon groups of up to 20 carbon atoms).

8. A method according to any one of claims 1 to 3 wherein the reducing system comprises a hydride source in the presence of a transition metal phthalocyanine or polyphthalocyanine in which the transition metal is iron, cobalt or a mixture of both and the hydride source is used in an amount of from 1 to 10 molar equivalents with respect to the compound of formula (2).

9. A method according to any one of claims 1 to 3 wherein the reducing system comprises a hydride source in the presence of a transition metal phthalocyanine or polyphthalocyanine in which the transition metal is iron, cobalt or a mixture of both and the transition metal phthalocyanine is used in an amount of from 1 to 10% molar equivalents with respect to the compound of formula (2).

10. The use of a reducing system selected from (i) hydrogen in the presence of a platinum oxide catalyst, or (ii) a hydride source in the presence of a transition metal phthalocyanine or polyphthalocyanine in which the transition metal is iron, cobalt or a mixture of both for effecting reduction preferentially at the 2-position of a compound according to the formula (2) defined in any one of claims 1 to 3 to form a corresponding compound of the formula (1) defined in any of claims 1 to 3.

11. The use according to claim 10 wherein the reducing system comprises a hydride source in the presence of a polyphthalocyanine polymer complexed with iron, cobalt or a mixture of both.

12. The use according to claim 10 wherein the reducing system comprises a hydride source in the presence of a transition metal phthalocyanine or polyphthalocyanine in which the transition metal is iron, cobalt or a mixture of both and the hydride source is selected from the group consisting of alkali metal tetrahydroborates, alkali metal hydrides, alkali metal tetrahydridoaluminates, alkali metal alkyl borohydrides (where the alkali metal is lithium, sodium or potassium, and up to three alkyl groups can be present, each alkyl group comprising branched chain, linear or cyclic carbon groups of up to 20 carbon atoms), alkali metal alkoxy borohydrides (where the alkali metal is lithium, sodium or potassium, and up to three alkoxy groups can be present, each alkoxy group comprising branched chain, linear or cyclic carbon groups of up to 20 carbon atoms), alkali metal alkyl hydridoaluminates (where the alkali metal is lithium, sodium or potassium, and up to three alkyl groups can be present, each alkyl group comprising branched chain, linear or cyclic carbon groups of up to 20 carbon atoms), and alkali metal alkoxyhydridoaluminates (where the alkali metal is lithium, sodium or potassium, and up to three alkoxy groups can be present , each alkoxy group comprising branched chain, linear or cyclic carbon groups of up to 20 carbon atoms).

## Patentansprüche

1. Verfahren zur Synthese einer Verbindung der folgenden Formel (1): 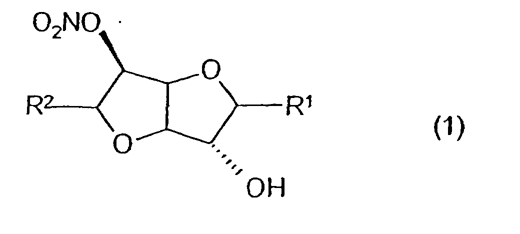 worin die R¹ und R² jeweils unabhängig voneinander aus H oder gegebenenfalls substituierten, unverzweigten oder verzweigten C₁-C₃₀-Alkyl-, C₁-C₃₀-Carboxyalkyl-, C₁-C₃₀-Sulfoxyalkyl-, C₁-C₃₀-Alkoxy-, C₃-C₃₀-Cycloalkyl-, C₃-C₃₀-Carboxycycloalkyl-, C₃-C₃₀-Sulfoxycycloalkyl-, C₃-C₃₀-Cycloalkoxy-, heterozyklischen, carboxyheterozyklischen, sulfoxyheterozyklischen, oxyheterozyklischen, C₃-C₃₀-Cycloalkenyl-, -Carboxycycloalkenyl-, -Sulfoxycycloalkenyl- oder -Cycloalkenoxy-, C₈-C₃₀-Cycloalkinyl-, -Carboxycycloalkinyl-, -Sulfoxycycloalkinyl- oder -Cycloalkinoxy-, C₂-C₃₀-Alkinyl-, -Carboxyalkinyl-, -Sulfoxyalkinyl- oder -Alkinoxygruppen, C₄-C₃₀-Aromaten-, -Carboxyaromaten-, -Sulfoxyaromaten oder -Aryloxy-, C₄-C₃₀-Heteroaromaten-, -Carboxyheteroaromaten-, -Sulfoxyheteroaromaten- oder -Heteroaryloxy-Gruppen ausgewählt sind, worin in jeder der Heteroatom-hältigen Gruppen das Heteroatom aus der aus O, S und N bestehenden Gruppe ausgewählt ist, und für den Fall, dass eine der oben genannten Gruppen substituiert ist, ein oder mehrere Substituenten vorhanden sind, die unabhängig voneinander aus der aus Halogen-, Cyano-, C₁-C₃₀-Alkyl-, C₂-C₃₀-Alkenyl-, C₄-C₃₀-Aromaten-, C₁-C₃₀-Ether-, C₁-C₃₀-Ester, C₁-C₃₀-Sulfonatester-, Nitro-, C₁-C₃₀-Keton-, C₁-C₃₀-Thioether- und/oder einer oder mehreren pharmazeutisch aktiven Gruppen bestehenden Gruppe ausgewählt sind; wobei das Verfahren das Behandeln einer Verbindung der folgenden Formel (2): 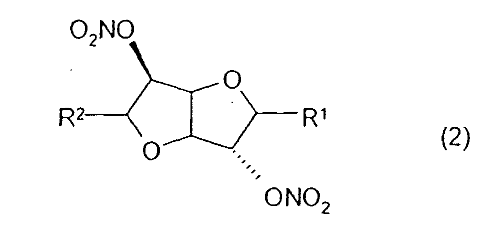 mit einem Reduktionssystem umfasst, das bevorzugt zur Reduktion der Verbindung der Formel (2) an der 2-Position wirksam ist, um die Verbindung der Formel (1) zu erzeugen, worin das Reduktionssystem (i) Wasserstoff in Gegenwart eines Platinoxidkatalysators oder (ii) eine Hydridquelle in Gegenwart eines Übergangsmetallphthalocyanins oder -polyphthalocyanins umfasst, worin das Übergangsmetall Eisen und/oder Cobalt ist.

2. Verfahren nach Anspruch 1, worin die pharmazeutische(n) Gruppe(n) aus der aus 2-Acetoxybenzoat, 2-N-(3'-Trifluormethylphenyl)aminobenzoat, (S)-6-Methoxy-α-methyl-2-naphthalinacetat und (S)-1-[N-[1-(Ethoxycarbonyl)-3-phenylpropyl]-L-alanyl]-L-prolincarboxylat bestehenden Gruppe ausgewählt ist bzw. sind.

3. Verfahren nach Anspruch 1, worin R¹ und R² beide Wasserstoff sind.

4. Verfahren nach einem der Ansprüche 1 bis 3, worin das Reduktionssystem Wasserstoff in Gegenwart eines Platinkatalysators umfasst und die Reaktion bei Raumtemperatur unter einer Wasserstoffgasatmosphäre von 5 bis 300 psi durchgeführt wird.

5. Verfahren nach Anspruch 3 oder Anspruch 4, worin die Reaktion in einem Lösungsmittel durchgeführt wird, das Dichlormethan/Methanol oder Dichlormethan/Ethanol umfasst.

6. Verfahren nach einem der Ansprüche 1 bis 3, worin das Reduktionssystem eine Hydridquelle in Gegenwart eines Polyphthalocyaninpolymers, das mit Eisen, Cobalt oder einem Gemisch der beiden komplexiert ist, umfasst.

7. Verfahren nach einem der Ansprüche 1 bis 3, worin das Reduktionssystem eine Hydridquelle in Gegenwart eines Übergangsmetallphthalocyanins oder -polyphthalocyanins umfasst, worin das Übergangsmetall Eisen, Cobalt oder ein Gemisch der beiden ist und die Hydridquelle aus der aus Alkalimetalltetrahydroboraten, Alkalimetallhydriden, Alkalimetalltetrahydridoaluminaten, Alkalimetallalkylborhydriden (worin das Alkalimetall Lithium, Natrium oder Kalium ist und bis zu drei Alkylgruppen vorhanden sein können, wobei jede Alkylgruppe verzweigte, lineare oder zyklische Kohlenstoffgruppen mit bis zu 20 Kohlenstoffatomen umfasst), Alkalimetallalkoxyborhydriden (worin das Alkalimetall Lithium, Natrium oder Kalium ist und bis zu drei Alkoxygruppen vorhanden sein können, wobei jede Alkoxygruppe verzweigte, lineare oder zyklische Kohlenstoffgruppen mit bis zu 20 Kohlenstoffatomen umfasst), Alkalimetallalkylhydridoaluminaten (worin das Alkalimetall Lithium, Natrium oder Kalium ist und bis zu drei Alkylgruppen vorhanden sein können, wobei jede Alkylgruppe verzweigte, lineare oder zyklische Kohlenstoffgruppen mit bis zu 20 Kohlenstoffatomen umfasst) und Alkalimetallalkoxyhydridoaluminaten (worin das Alkalimetall Lithium, Natrium oder Kalium ist und bis zu drei Alkoxygruppen vorhanden sein können, wobei jede Alkoxygruppe verzweigte, lineare oder zyklische Kohlenstoffgruppen mit bis zu 20 Kohlenstoffatomen umfasst) bestehenden Gruppe ausgewählt ist.

8. Verfahren nach einem der Ansprüche 1 bis 3, worin das Reduktionssystem eine Hydridquelle in Gegenwart eines Übergangsmetallphthalocyanins oder -polyphthalocyanins umfasst, worin das Übergangsmetall Eisen, Cobalt oder ein Gemisch der beiden ist und die Hydridquelle in Bezug auf die Verbindung der Formel (2) in einer Menge von 1 bis 10 Moläquivalenten eingesetzt wird.

9. Verfahren nach einem der Ansprüche 1 bis 3, worin das Reduktionssystem eine Hydridquelle in Gegenwart eines Übergangsmetallphthalocyanins oder -polyphthalocyanins umfasst, worin das Übergangsmetall Eisen, Cobalt oder ein Gemisch der beiden ist und das Übergangsmetallphthalocyanin in Bezug auf die Verbindung der Formel (2) in einer Menge von 1 bis 10 Moläquivalent-Prozenten eingesetzt wird.

10. Verwendung eines Reduktionssytems, das aus (i) Wasserstoff in Gegenwart eines Platinoxidkatalysators oder (ii) einer Hydridquelle in Gegenwart eines Übergangsmetallphthalocyanins oder -polyphthalocyanins, worin das Übergangsmetall Eisen, Cobalt oder ein Gemisch der beiden ist, ausgewählt ist, um bevorzugt an der 2-Position einer Verbindung der Formel (2), wie in einem der Ansprüche 1 bis 3 definiert, eine Reduktion durchzuführen, um eine entsprechende Verbindung der Formel (1), wie in einem der Ansprüche 1 bis 3 definiert, herzustellen.

11. Verwendung nach Anspruch 10, worin das Reduktionssystem eine Hydridquelle in Gegenwart eines Polyphthalocyaninpolymers umfasst, das mit Eisen, Cobalt oder einem Gemisch der beiden komplexiert ist.

12. Verwendung nach Anspruch 10, worin das Reduktionssystem eine Hydridquelle in Gegenwart eines Übergangsmetallphthalocyanins oder -polyphthalocyanins umfasst, worin das Übergangsmetall Eisen, Cobalt oder ein Gemisch der beiden ist und die Hydridquelle aus der aus Alkalimetalltetrahydroboraten, Alkalimetallhydriden, Alkalimetalltetrahydridoaluminaten, Alkalimetallalkylborhydriden (worin das Alkalimetall Lithium, Natrium oder Kalium ist und bis zu drei Alkylgruppen vorhanden sein können, wobei jede Alkylgruppe verzweigte, lineare oder zyklische Kohlenstoffgruppen mit bis zu 20 Kohlenstoffatomen umfasst), Alkalimetallalkoxyborhydriden (worin das Alkalimetall Lithium, Natrium oder Kalium ist und bis zu drei Alkoxygruppen vorhanden sein können, wobei jede Alkoxygruppe verzweigte, lineare oder zyklische Kohlenstoffgruppen mit bis zu 20 Kohlenstoffatomen umfasst), Alkalimetallalkylhydridoaluminaten (worin das Alkalimetall Lithium, Natrium oder Kalium ist und bis zu drei Alkylgruppen vorhanden sein können, wobei jede Alkylgruppe verzweigte, lineare oder zyklische Kohlenstoffgruppen mit bis zu 20 Kohlenstoffatomen umfasst) und Alkalimetallalkoxyhydridaluminaten (worin das Alkalimetall Lithium, Natrium oder Kalium ist und bis zu drei Alkoxygruppen vorhanden sein können, wobei jede Alkoxygruppe verzweigte, lineare oder zyklische Kohlenstoffgruppen mit bis zu 20 Kohlenstoffatomen umfasst) bestehenden Gruppe ausgewählt ist.

## Revendications

1. Procédé de synthèse d'un composé de la formule (1) suivante, 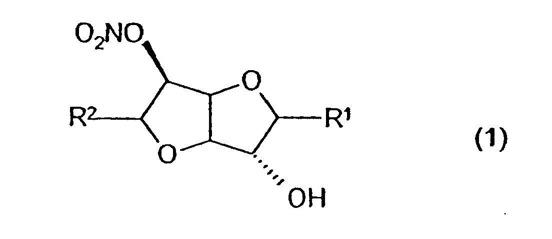 dans laquelle chacun des R¹ et R² est indépendamment choisi parmi H ou un alkyle en C₁-C₃₀, un carboxyalkyle en C₁-C₃₀, un sulfoxyalkyle en C₁-C₃₀, un alcoxyle en C₁-C₃₀, un cycloalkyle en C₃-C₃₀, un carboxycycloalkyle en C₃-C₃₀, un sulfoxycycloalkyle en C₃-C₃₀, un cycloalcoxy en C₃-C₃₀, un hétérocyclique, un carboxyhétérocyclique, un sulfoxyhétérocyclique, un oxyhétérocyclique, un cycloalcényle, carboxycycloalcényle, sulfoxycycloalcényle ou cycloalcénoxy en C₃-C₃₀, un cycloalkynyle, un carboxycycloalkynyle, un sulfoxycycloalkynyle ou un cycloalkynoxy en C₈-C₃₀, un groupement alkynyle, carboxyalkynyle, sulfoxyalkynyle ou alkynoxy en C₂-C₃₀, un groupement aromatique, carboxyaromatique, sulfoxyaromatique ou aryloxy en C₄-C₃₀, un groupement hétéroaromatique, carboxyhétéroaromatique, sulfoxyhétéroaromatique ou hétéroaryloxy en C₄-C₃₀, tous à chaîne droite ou ramifiée éventuellement substituée où dans l'un quelconque desdits groupements contenant des atomes hétéros, l'atome hétéro est choisi dans le groupe se composant de O,S, et N et, dans le cas de l'un quelconque des groupements mentionnés ci-dessus qui est substitué, il existe un ou plusieurs substituants choisis de manière indépendante dans le groupe se composant des groupes halogènes, cyano, alkyle en C₁-C₃₀, alcényle en C₂-C₃₀, aromatique en C₄-C₃₀, éther en C₁-C₃₀, ester en C₁-C₃₀, estersulfonate en C₁-C₃₀, nitro, cétone en C₁-C₃₀, thioéther en C₁-C₃₀ et / ou un ou plusieurs groupes pharmaceutiquement actif ; le procédé comprenant le traitement d'un composé de la formule (2) suivante : 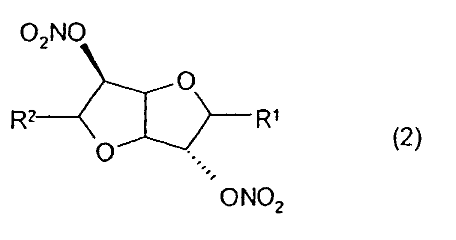 avec un système réducteur efficace pour réduire préférentiellement le composé de formule (2) à la position 2 pour produire le composé de formule (1), où le système réducteur comprend (i) de l'hydrogène en présence d'un catalyseur à oxyde de platine, ou (ii) une source d'hydrure en présence d'une phtalocyanine ou d'une polyphtalocyanine d'un métal de transition dans laquelle le métal de transition est le fer et/ou le cobalt.

2. Un procédé selon la revendication 1 dans lequel le ou chaque groupe pharmaceutiquement actif est choisi dans le groupe se composant de 2-acétoxybenzoate, de 2-N-(3'-trifluorométhylphényl) aminobenzoate, de (S)-6-méthoxy-α-méthyl-2-naphtalèneacétate et de (S)-1-[N-[1-(éthoxycarbonyl)-3-phénylpropyl]-L-alanyl]-L-prolinecarboxylate.

3. Un procédé selon la revendication 1 dans lequel R¹ et R² sont tous deux l'hydrogène.

4. Un procédé selon une quelconque des revendications 1 à 3 dans lequel le système réducteur comprend de l'hydrogène en présence d'un catalyseur au platine et la réaction est mise en oeuvre à température ambiante sous une atmosphère d'hydrogène gazeux de 5 à 300 psi.

5. Un procédé selon la revendication 3 ou la revendication 4 dans lequel la réaction est mise en oeuvre dans un solvant comprenant du dichlorométhane / méthanol ou du dichlorométhane / ethanol.

6. Un procédé selon une quelconque des revendications 1 à 3 dans lequel le système réducteur comprend une source d'hydrure en présence d'un polymère de polyphtalocyanine complexé avec du fer, du cobalt ou un mélange des deux.

7. Un procédé selon une quelconque des revendications 1 à 3 dans lequel le système réducteur comprend une source d'hydrure en présence d'une phtalocyanine ou d'un polyphtalocyanine de métal de transition où le métal de transition est le fer, le cobalt ou un mélange des deux et la source d'hydrure est choisie dans le groupe se composant de tetrahydroborates de métal alcalin, d'hydrures de métal alcalin, de tetrahydridolaluminates de métal alcalin, de borohydrures alkyliques de métal alcalin (où le métal alcalin est le lithium, le sodium ou le potassium et où jusqu'à trois groupes alkyles peuvent être présents, chaque groupe alkyle comprenant des groupes carbonés linéaires, cycliques ou à chaînes ramifiées ayant jusqu'à vingt atomes de carbones), des alcoxy borohydrures de métal alcalin (où le métal alcalin est le lithium, le sodium ou le potassium et où jusqu'à trois groupes alcoxy peuvent être présents, chaque groupe alcoxy comprenant des groupes carbonés linéaires, cycliques ou à chaînes ramifiées ayant jusqu'à vingt atomes de carbone), des hydridoaluminates alkyliques de métal alcalin (où le métal alcalin est le lithium, le sodium, ou le potassium, et où jusqu'à trois groupes alkyles peuvent être présents, chaque groupe alkyle comprenant des groupes carbonés cycliques, linéaires ou à chaînes ramifiées ayant jusqu'à vingt atomes de carbone), et des alcoxyhydridoaluminates de métal alcalin (où le métal alcalin est le lithium, le sodium, ou le potassium, et où jusqu'à trois groupes alcoxy peuvent être présents, chaque groupe alcoxy comprenant des groupes carbonés linéaires, cycliques ou à chaînes ramifiées ayant jusqu'à vingt atomes de carbone).

8. Un procédé selon une quelconque des revendications 1 à 3 dans lequel le système réducteur comprend une source d'hydrure en présence d'une phtalocyanine ou polyphtalocyanine d'un métal de transition où le métal de transition est le fer, le cobalt ou un mélange des deux et où la source d'hydrure est utilisée dans une quantité allant de 1 à 10 équivalents molaires par rapport au composé de formule (2).

9. Un procédé selon l'une quelconque des revendications 1 à 3 dans lequel le système réducteur comprend une source d'hydrure en présence d'une phtalocyanine ou d'une polyphtalocyanine d'un métal de transition où le métal de transition est le fer, le cobalt ou un mélange des deux et où la phtalocyanine d'un métal de transition est utilisé dans une quantité allant de 1 à 10% d'équivalent molaire par rapport au composé de formule (2).

10. L'utilisation d'un système réducteur choisi parmi (i) l'hydrogène en présence d'un catalyseur à l'oxyde de platine, ou (ii) une source d'hydrure en présence d'une phtalocyanine ou d'une polyphtalocyanine d'un métal de transition où le métal de transition est le fer, le cobalt ou un mélange des deux pour effectuer une réduction préférentiellement à la position (2) d'un composé selon la formule 2 défini dans une quelconque des revendications 1 à 3 pour former un composé correspondant de la formule (1) défini dans une quelconque des revendications 1 à 3.

11. L'utilisation selon la revendication 10 dans laquelle le système réducteur comprend une source d'hydrure en présence d'un polymère de polyphtalocyanine complexé avec du fer, du cobalt ou un mélange des deux.

12. L'utilisation selon la revendication 10 dans laquelle le système réducteur comprend une source d'hydrure en présence d'une phtalocyanine ou d'une polyphtalocyanine d'un métal de transition dans laquelle le métal de transition est le fer, le cobalt ou un mélange des deux et où la source d'hydrure est choisie dans le groupe se composant de tetrahydroborates de métal alcalin, d'hydrures de métal alcalin, de tetrahydridoaluminates de métal alcalin, de borohydrures alkyliques de métal alcalin (où le métal alcalin est le lithium, le sodium ou le potassium, et où jusqu'à trois groupes alkyles peuvent être présents, chaque groupe alkyle comprenant des groupes carbonés linéaires, cycliques ou à chaînes ramifiées ayant jusqu'à vingt atomes de carbone), des alcoxy borohydrures d'un métal alcalin (où le métal alcalin est le lithium, le sodium ou le potassium, et où jusqu'à trois groupes alcoxy peuvent être présents, chaque groupe alcoxy comprenant des groupes carbonés linéaires, cycliques ou à chaînes ramifiées ayant jusqu'à vingt atomes de carbone), des hydridoaluminates alkyliques de métal alcalin (où le métal alcalin est le lithium, le sodium ou le potassium, et où jusqu'à trois groupes alkyles peuvent être présents, chaque groupe alkyle comprenant des groupes linéaires, cycliques ou à chaînes ramifiées ayant jusqu'à vingt atomes de carbone), et des alcoxy hydridoaluminates d'un métal alcalin (où le métal alcalin est le lithium, le sodium ou le potassium, et où jusqu'à trois groupes alcoxy peuvent être présents, chaque groupe alcoxy comprenant des groupes carbonés linéaires, cycliques ou à chaînes ramifiées ayant jusqu'à vingt atomes de carbone).
